(19) Europäisches Patentamt / European Patent Office / Office européen des brevets

(11) **EP 4 472 614 B1**

(12) **EUROPEAN PATENT SPECIFICATION**

(45) Date of publication and mention of the grant of the patent:
**04.03.2026 Bulletin 2026/10**

(21) Application number: **23703707.2**

(22) Date of filing: **31.01.2023**

(51) International Patent Classification (IPC):
***A61K 9/00*** *(2006.01)* ***A61K 9/08*** *(2006.01)*
***A61K 31/00*** *(2006.01)* ***A61K 47/26*** *(2006.01)*
***A61P 27/02*** *(2006.01)* ***A61K 47/36*** *(2006.01)*

(52) Cooperative Patent Classification (CPC):
(C-Sets available)
**A61K 9/0048; A61K 9/08; A61K 31/05; A61K 31/702; A61K 31/728; A61K 47/26; A61K 47/36; A61P 27/02;** A61J 1/067 (Cont.)

(86) International application number:
**PCT/EP2023/052367**

(87) International publication number:
**WO 2023/148180 (10.08.2023 Gazette 2023/32)**

(54) **OPHTHALMIC COMPOSITION COMPRISING RESVERATROL FOR TREATING DRY EYE SYNDROME**

OPHTHALMISCHE ZUSAMMENSETZUNG MIT RESVERATROL ZUR BEHANDLUNG DES TROCKENEN-AUGEN-SYNDROMS

COMPOSITION OPHTALMIQUE COMPRENANT DU RESVÉRATROL POUR TRAITER LE SYNDROME DE L’OEIL SEC

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Validation States:
**MA TN**

(30) Priority: **01.02.2022 EP 22305113**

(43) Date of publication of application:
**11.12.2024 Bulletin 2024/50**

(73) Proprietor: **Laboratoires THEA**
**63100 Clermont-Ferrand (FR)**

(72) Inventors:
- **LEBRETON, Luc**
  **63110 BEAUMONT (FR)**
- **ALLAN, Fanny**
  **63111 MUR SUR ALLIER (FR)**
- **NAJI, Mohammed**
  **63800 COURNON D’AUVERGNE (FR)**

(74) Representative: **Ipsilon**
**Le Contemporain**
**50 Chemin de la Bruyère**
**69574 Dardilly Cedex (FR)**

(56) References cited:
**WO-A2-2009/084069 CN-A- 113 286 577**
**KR-B1- 101 433 152 US-A1- 2021 046 018**

- **CARETTI L ET AL: "Efficacy of carbomer sodium hyaluronate trehalose vs hyaluronic acid to improve tear film instability and ocular surface discomfort after cataract surgery", vol. Volume 13, 1 July 2019 (2019-07-01), pages 1157 - 1163, XP055943121, Retrieved from the Internet <URL:https://www.dovepress.com/getfile.php?fileID=51086> [retrieved on 20220715], DOI: 10.2147/OPTH.S208256**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61K 31/05, A61K 2300/00;**
**A61K 31/702, A61K 2300/00;**
**A61K 31/728, A61K 2300/00**

## Description

### FIELD OF THE INVENTION

This invention concerns an ophthalmic composition for topical application, intended notably to treat dry eyes. Such a composition combines hyaluronic acid or a salt thereof, trehalose and resveratrol.

### STATE OF THE ART

Dry eye syndrome, also known as ocular dryness or dry keratoconjunctivitis or keratoconjunctivitis sicca (KCS), causes discomfort of the eye, visual disturbance and tear film instability, accompanied by itching, tingling and/or burning sensations.

It is a multifactorial disease Dry eyes can result from abnormal tear glands, inflammation of the eyelids, inflammation of the eyelids, allergy-related eye inflammation, refractive surgery (including laser surgery), deficiency of certain lipids in the daily diet, prolonged wearing of contact lenses, hormonal alteration, autoimmune disease, or side effects of certain drugs.

The incidence of dry eyes increases with age, and aging is associated with increased oxidative stress. Furthermore, environmental factors are implicated in dry eye pathophysiology such as exposure to pollution, blue light, ultraviolet (UV) radiation and ozone. These factors increase oxidative stress and ocular surface inflammation. Oxidative stress generates reactive oxygen species (ROS) that can cause deleterious alterations in deoxyribonucleic acid, lipid, and protein of corneal and conjunctival epithelial cells that can lead to ocular surface diseases (OSD) such as dry eye disease (DED).

In a preventive manner and/or at the early stage of some ocular diseases, it is recommended to daily take a food supplement such as the one disclosed in WO2016/151269 containing vitamins, oligoelements, carotenoids, omega 3 fatty acids and resveratrol.

In the treatment of dry eyes, the application of artificial tears, also known as lubricating or moisturizing eye drops, provides local but short-lasting relief. Artificial tears of low to medium viscosity are generally based on polyvinyl alcohols or cellulosic derivatives, while those of higher viscosity contain carbomers or hyaluronic acid.

Hyaluronic acid (HA) is used in eye drops for the moisturizing of the cornea wherein its "barrier" effect with respect to friction reduces the pain caused by dry eyes. It forms a transparent, lubricating and wetting film on the surface of the eye, protecting the cornea from drying out. These properties offer effective relief of functional signs and allow fewer daily instillations for the patient.

Studies showed that HA eye drops are very well accepted by even the driest eyes. After instillation, a slight discomfort of between a few seconds and a few minutes may appear. Vision becomes slightly blurred. It is therefore better not to use the product "in an emergency", before taking the road. The discomfort disappears very quickly and the product persists for much longer than simple eye drops. The action time depends on how dry the eyes are. As a general rule, simple eye drops are effective for 10 to 15 minutes while the action of hyaluronic acid lasts rather for 45 minutes to 1 hour.

The conclusion is that hyaluronic acid-based eye drops are effective for light to moderate dry eye. Patients with severe eye dryness may also find it worthwhile but must generally turn to different products, such as immunosuppressive eye drops such as cyclosporine.

To improve the efficacy of hyaluronic acid-based eye drops, document WO2015/136186 has proposed to combine HA, advantageously of low molecular weight (100 to 800 kDa) with an oligosaccharide, advantageously a diholoside, in a topical formulation having increased persistence i.e. retention time on ocular surface.

Besides, VisuFarma company has launched products to improve the oxidative component of DED: Visu XL® eye drop product contains the antioxidant Coenzyme Q (Ubiquinone Q10) in association with vitamin E TPGS, and crosslinked sodium hyaluronate.

Topical ophthalmic compositions, which comprise resveratrol and hyaluronic acid, have also been disclosed, see e.g. document WO 2009/084069.

However, there is a persistent need to develop ophthalmic formulations having improved efficacy for treating ocular surface diseases, especially DED.

### DESCRIPTION OF THE INVENTION

The present invention, as defined by the claims, proposes a composition useful in the treatment of dry eyes, by combining a lubricant polymer, in particular hyaluronic acid already known for its use in this application, and an oligosaccharide, both improving the remanence and bioavailability of resveratrol which in turns prevents the ocular surface from the oxidative stress induced by external environment or induced by dry eye disease by preventing the production and inhibiting free radicals, the root cause of tissue damage.

Definitions

Unless otherwise defined, all technical and scientific terms used therein have the same meaning as commonly understood by one of ordinary skill in the art. The terminology used in the description is for the purpose of describing particular embodiments only and is not intended to be limiting.

The articles "a" and "an" are used herein to refer to one or to more than one (*i.e.,* to at least one) of the grammatical object of the article. By way of example, "an element" means one element or more than one element.

"About" or "approximately" as used herein when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

The term "abnormal" when used in the context of organisms, tissues, cells or components thereof, refers to those organisms, tissues, cells or components thereof that differ in at least one observable or detectable characteristic (e.g., age, treatment, time of day, etc.) from those organisms, tissues, cells or components thereof that display the "normal" (expected) respective characteristic. Characteristics, which are normal or expected for one cell or tissue type, might be abnormal for a different cell or tissue type.

The terms "patient," "subject," "individual," and the like are used interchangeably herein, and refer to any animal, or cells thereof whether *in vitro* or *in situ,* amenable to the methods described herein. A subject can be an animal, advantageously a mammal. In certain nonlimiting embodiments, the patient, subject or individual is a human.

A "disease" or a "pathology" is a state of health of a subject wherein the subject cannot maintain homeostasis, and wherein if the disease is not ameliorated then the subject's health continues to deteriorate. In contrast, a "disorder" in a subject is a state of health in which the subject is able to maintain homeostasis, but in which the subject's state of health is less favorable than it would be in the absence of the disorder. Left untreated, a disorder does not necessarily cause a further decrease in the subject's state of health.

A disease or disorder is "alleviated", "ameliorated" or "relieved" if the severity of a symptom of the disease or disorder, the frequency with which such a symptom is experienced by a patient, or both, is reduced. This also includes halting progression of the disease or disorder. A disease or disorder is "cured" if the severity of a symptom of the disease or disorder, the frequency with which such a symptom is experienced by a patient, or both, is eliminated.

A "therapeutic" treatment is a treatment administered to a subject who exhibits signs of pathology, for the purpose of diminishing or eliminating those signs. A "prophylactic" treatment is a treatment administered to a subject who does not exhibit signs of pathology or has not be diagnosed for the pathology yet, for the purpose of preventing or postponing the occurrence of those signs.

As used herein, "treating a disease or disorder" means reducing the frequency or severity of at least one sign or symptom of a disease or disorder experienced by a subject. Disease and disorder are used interchangeably herein in the context of treatment.

In the context of the invention, an "active principle" or "active compound" or "active agent" or "active substance" or "active constituent" or "active" is the molecule or ingredient in a pharmaceutical drug that is biologically active.

An "effective amount" of a compound is that amount of compound which is sufficient to provide a beneficial effect to the subject to which the compound is administered. The phrase "therapeutically effective amount", as used herein, refers to an amount that is sufficient or effective to prevent or treat (delay or prevent the onset of, prevent the progression of, inhibit, decrease or reverse) a disease or condition, including alleviating symptoms of such diseases. An "effective amount" of a delivery vehicle is that amount sufficient to effectively bind or deliver a compound.

Thus, and in a first aspect, this application describes an ophthalmic composition, as defined by the claims, comprising a lubricant polymer, an oligosaccharide, and an antioxidant. According to the invention, the composition contains hyaluronic acid or one of its salts, advantageously with a molecular weight between 100 and 800 kDa; trehalose; and resveratrol.

In the context of the application, a lubricating polymer, advantageously moisturizing, is defined as being capable of forming a transparent film on the surface of the eye, in particular because of its water retention capacity.

According to another preferred embodiment, this polymer is not a cellulose derivative, in particular this polymer is not carboxymethylcellulose (CMC).

According to the invention, said glycosaminoglycan is hyaluronic acid, or pharmaceutically acceptable salts

thereof

Hyaluronic acid, which is characterized by the absence of O-sulphation, is used in the invention, particularly in the form of sodium hyaluronate. Sodium hyaluronate, naturally found in the human eye, holds water to hydrate and lubricate the surface of the eye. It keeps the solution on the eye surface giving long lasting relief and cutting down the healing time of the ocular surface.

The polymer used will in fact determine the viscosity of the ophthalmic composition. As this viscosity depends both on the polymer concentration and the molecular weight of this polymer, these two factors are adjusted so that the composition has an appropriate viscosity not causing any discomfort (at the time of instillation or after) to the user.

Thus and in an appropriate manner, particularly for hyaluronic acid, the centesimal concentration of the composition in lubricant polymer is less than or equal to 0.5%, advantageously to 0.2%. In other words, the weight of the lubricant polymer advantageously represents at most 0.5 g or even 0.2 g in 100 ml of the composition. In addition, and for efficiency purposes, the centesimal concentration of lubricant polymer is advantageously equal to or higher than 0.05% (0.05g/100ml) or even 0.1% (0.1g/100ml). In other words, and according to a preferred embodiment, the lubricant polymer, preferably HA or one of its salts such as sodium hyaluronate, represents between 0.05 and 0.5 g per 100 ml of the composition, advantageously between 0.1 and 0.2 g/100 ml, for example 0.15 g/100 ml.

As already mentioned, the polymer is preferably in non-reticulated form. In addition, for hyaluronic acid, the preferred molecular weight (MW) is between 100 kDa and 800 kDa (medium MW).

Another component of the formulation of the invention is trehalose as an oligosaccharide Oligosaccharides, also called oligoholosides or oligosides, are oligomers formed by a number n of ose (or monosaccharide) units, with n generally ranging from 2 to 10. Oligosides can be linear, branched or cyclic.

It is therefore not a polyol of the glycerol or glycerine type.

Note that these oligosaccharides may have intrinsic properties of interest in the ophthalmic field, in particular an anti-inflammatory, anti-apoptotic, or osmolarity regulation activity. Besides and as shown in the examples, it would contribute to the stability and activity of the composition according to the invention.

According to the invention, said oligosaccharide is trehalose. Trehalose is a natural substance present in many plants and animals, which can survive in extremely dry conditions. It provides protective and hydration properties.

In another embodiment, the centesimal concentration of the oligosaccharide is less than or equal to 5% by weight of the composition, advantageously to 4%. In other words, the content of the oligosaccharide advantageously represents at most 5 g or even 4 g in 100 ml of the composition. In addition, and for efficiency purposes, the centesimal concentration of the oligosaccharide is advantageously higher or equal to 0.5% (0.5 g/100ml) or even 2% (2 g/100ml). In other words, and according to a preferred embodiment, the oligosaccharide, preferably trehalose, represents between 0.5 and 5 g per 100 ml of the composition, advantageously between 2 and 4 g/100 ml, for example 3 g/100 ml.

A third ingredient of the composition according to the invention is resveratrol. Resveratrol is a natural product known for its antioxidant and anti-free-radical properties.

It is a polyphenolic compound, derived from stilbene, of the formula:

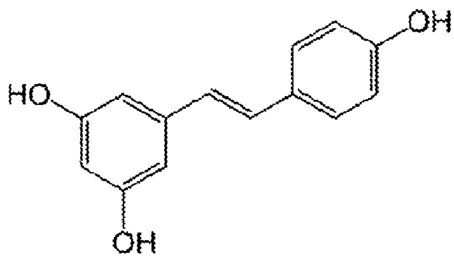

There are two isomers, but the *trans* form is predominantly active. In the description that follows, the term "resveratrol" may therefore be used instead and in place of *trans*-resveratrol.

A high concentration of resveratrol, ideally 0.1% w/v, is preferred for a potent antioxidant activity but a concentration that ensure good efficiency and ocular tolerance is acceptable. Then, resveratrol advantageously represents between 0.01 and 0.1 g in 100 ml of the composition, advantageously between 0.04 and 0.06 g/100 ml, even more advantageously 0.05 g/100 ml. In other words, the concentration in resveratrol can be e.g. equal to 0.01, 0.02, 0.03, 0.04, 0.05, 0.06, 0.07, 0.08, 0.09 or 0.1 g/100 ml. According to a specific embodiment, the concentration in resveratrol is inferior or equal to 0.08 g/100 ml

According to a preferred embodiment, the composition of the invention is in a liquid form, preferably a solution. In that context, solution generally refers to a liquid pharmaceutical composition in which compound(s) are at least partially dissolved, preferably fully dissolved, and which can be administered as a liquid. According to a specific embodiment and in relation to its ocular instillation, the composition is advantageously an aqueous solution i.e. a solution containing water as a main vehicle.

Taking into consideration the hydrophobic nature of resveratrol, the composition of the invention advantageously further contains a solubilizing agent.

According to a preferred embodiment, the solubilizing agent is capable of solubilizing resveratrol in water. According to another preferred embodiment, the solubilizing agent is compatible with ocular administration, i.e. does not display any toxicity at the administered concentration.

Such a solubilizing agent is advantageously macrogol hydroxystearate, more advantageously macrogolglycerol hydroxystearate (polyoxyl 40 hydrogenated castor oil or hydrogenated ethoxylated castor oil or PEG-40 Hydrogenated Castor Oil or Cremophor® RH40 or Kolliphor® RH40; CAS number 61788-85-0).

Other potential candidates are e.g.:

- polyoxyl 15 hydroxystearate (macrogol 15 hydroxystearate or Kolliphor® HS15 or Solutol® HS15; CAS number 70142-34-6);
- polysorbate 20 (CAS number 9005-64-5), polysorbate 60 (CAS number 9005-67-8) or polysorbate 80 (CAS number 9005-65-6);
- macrogolglycerol ricinoleate (or polyoxyl 35 castor oil or PEG-35 Castor Oil or Cremophor® EL or Kolliphor® EL; CAS number 61791-12-6);
- Vitamin E TPGS (TPGS or Vitamin E polyethylene glycol succinate or D-$\alpha$-Tocopherol polyethylene glycol (1000) succinate; CAS number 9002-96-4).

The concentration of the solubilizing agent in the solution is typically between 0.1 and 20% (w/v), advantageously between 0.5 and 5% (w/v), more advantageously 0.5% or 1% (w/v).

Of course, the composition according to the invention may also further contain at least one additive component chosen from the group of non-ionic isotonic agents, antioxidants and/or buffer systems.

Preferably, the composition according to the invention has a slightly acid pH, advantageously inferior to 7, but advantageously superior to 5 or even 5.5. The pH of the composition can be 5.6, 5.7, 5.8, 5.9, 6, 6.1, 6.2, 6.3, 6.4, 6.5, 6.6, 6.7, 6.8 or 6.9. It is advantageously comprised between 5.5 and 6.9, more advantageously equal to 6.0.

A slightly acidic pH can be obtained by adding an acid, e.g. hydrochloric acid (HCl).

In relation to the desired slightly acid pH which prevents the conversion of phenol to phenolate and then the apparition of an undesired yellow colour, a suitable buffer is advantageously selected in the group consisting of citrate, borate and phosphate, more advantageously citrate. As an example, trisodium citrate, $2H_2O$ can be used. The adequate quantity thereof can be determined by the skilled person, e.g. 0.588 g/ 100 ml for a 20 mM solution.

According to a specific embodiment, such a buffer is not a Tris buffer such as Trometamol present in the Thealoz® duo formulation disclosed in WO2015/136186.

In an advantageous manner, the composition according to the invention has a viscosity adapted to topical ophthalmic application.

Thus, the composition according to the invention preferably has a viscosity lower than or equal to 50 mPa.s (cP), or even lower than or equal to 20, 15, 10 or even 5 mPa.s. By adaptation, it is between 2 and 50 mPa.s, advantageously between 2 and 15 mPa.s, even more advantageously between 2 and 10 mPa.s, for example in the order of 5 mPa.s. These values correspond to the values measured with a BROOKFIELD RVDV III mobile rotating viscometer at 25°C.

In practice, this viscosity is advantageously close to the viscosity of human tears, estimated at about 6.5 mPa.s at 20°C and with a velocity gradient close to zero. The application of the composition according to the invention thus does not cause any sensation of discomfort or irritation. Indeed, it has been determined that an easy and unhindered elimination is only possible if the viscosity of an ophthalmic composition is less than 50 mPa.s.

According to another particular embodiment, it corresponds to a hypotonic to isotonic formulation, with an osmolality advantageously between 170 and 350 mosmol/kg, more advantageously between 200 and 300 mosmol/kg, e.g. around 250 mosmol/kg. This characteristic can be obtained, for example, by adding sodium chloride (NaCl). Oligosaccharide, at the concentration used, can also contribute to the regulation of the tonicity of the solution.

According to another embodiment, the composition according to the invention is free of antimicrobial preservatives, in particular quaternary ammonium preservatives, in particular benzalkonium chloride (BAK) known for its deleterious effects on the ocular surface. In other words, it is advantageously a so-called unpreserved composition or preservative free composition.

In the context of the invention, "anti-microbial agent or anti-microbial preservative or preservative agent" refers to a preservative with anti-microbial properties, i.e. a compound capable of protecting the ophthalmic composition against possible microbial contamination.

Quaternary ammonium compounds, in particular benzalkonium chloride (BAK) or Polyquad®, possibly in association with EDTA, are generally used as preservatives. Other preservatives are for example:

- guanidine-based preservatives (PHMB, chlorhexidine);

- benzyl alcohol;
- parabens (methyl, ethyl, propyl, ...) ;
- mercury-based preservatives, such as thimerosal;
- chlorobutanol;
- benzethonium chloride;
- oxidizing preservatives (Purite, ...).

In case of an unpreserved composition, it is advantageously presented in single-use vials (unidoses) or in a preservative free multidose vial, such as Abak®, Novelia®, Comod® vials or equivalent, i.e. vials allowing the delivery of preservative-free eye drops for several days.

The invention therefore also concerns a single-use (single-dose) or multi-dose vial adapted to unpreserved compositions comprising the ophthalmic composition previously described. As known in this field, such vials, containers or drop dispensers can be made of low density polyethylene (LDPE), advantageously of European Pharmacopoeia (EP) quality, and containing no additives.

Another aspect of the invention concerns the use of such a composition in the treatment of eye disorders and pathologies. In other words, the invention concerns a method of treatment of ocular diseases comprising administering the composition of the invention.

Thus, the combination of polymeric lubricant, oligosaccharide and resveratrol can be used in the context of dry eye disease (DED or dry eye syndrome (DES)), especially in case of mild to moderate forms, and possibly for the treatment of eye inflammation.

Further ocular inflammatory conditions are e.g. non-infectious uveitis (anterior, intermediate, posterior, pan), non-infectious conjunctivitis, iritis, or scleritis.

More generally, the ophthalmic composition of the invention can be used to reduce the effects of oxidative stresses at the eye level and for the treatment of the pathological manifestations related to apoptosis on the part of corneal keratocytes.

Hence, a further aspect of the invention is the use of said composition for the treatment of degenerative pathologies comprising ischemic opticopathy, senile opacity of lenses, cataract, uveitis, as well as of heredofamilial, dysmetabolic and senile age-related degenerative pathologies, and of neurodegenerative pathologies of the retina and of the optical nerve comprising glaucoma, senile macular degeneration, retinitis pigmentosa, Stargardt disease, vitelliform macular cysts, cones dystrophy, proliferative diabetic retinopathy, hypertensive retinopathy, retinal detachment, retinoblastoma, as well as neuritis and optical neuropathies of toxic, inflammatory and degenerative origin.

A composition of the invention may contain further active agent(s) to treat the same pathology or another ocular disease, e.g. an ocular infections, an ocular allergy or glaucoma.

In other words, the composition according to the invention may contain one or more other active agents, in a therapeutically effective amount. These may include antibiotics, for example, antibacterials, antifungals, antivirals, immunosuppressants, anti-inflammatory or anti-glaucomatous agents such as prostaglandins, beta-blockers, carbonic anhydrase inhibitors or alpha-adrenergic agonists.

Such an active agent can be chosen from the following group: aceclidine, acetazolamide, aciclovir, anecortave, apraclonidine, atropine, azapentacene, azelastine, bacitracine, befunolol, betamethasone, betaxolol, bimatoprost, brimonidine, brinzolamide, carbachol, carteolol, celecoxib, chloramphenicol, chlortetracycline, ciprofloxacin, cromoglycate, cyclopentolate, cyclosporine, dapiprazole, demecarium, dexamethasone, diclofenac, dichlorphenamide, dipivefrine, dorzolamide, echothiophale, emedastine, epinastine, epinephrine, erythromycin, ethoxzolamide, eucatropine, fludrocortisone, fluorometholone, flurbiprofen, fomivirsen, framycetine, ganciclovir, gatifloxacin, gentamycin, homatropin, hydrocortisone, idoxuridin, indomethacin, isoflurophate, ketorolac, ketotifen, latanoprost, levobetaxolol, levobunolol, levocabastine, levofloxacin, lodoxamide, loteprednol, medrysone, methazolamide, metipranolol, moxifloxacin, naphazoline, natamycin, nedocromil, neomycin, norfloxacin, ofloxacin, olopatadin, oxymetazoline, pemirolast, pegaptanib, phenylephrine, physostigmine, pilocarpine, pindolol, pirenoxin, polymyxin B, prednisolone, proparacaine, ranibizumab, rimexolone, scopolamine, scopolamine, sezolamide, squalamine, sulfacetamide, suprofen, tetracaine, tetracycline, tetrahydrozoline, tetryzoline, timolol, tobramycin, travoprost, triamcinulone, trifluoromethazolarnide, trifluridine, trimethoprim, tropicamide, unoprostone, vidarbine, xylometazoline, pharmaceutically acceptable salts, or combinations thereof.

A composition according to the invention can be used in humans as well as in animals. A large spectrum of individuals can be treated: adults, children, pregnant or breastfeeding women, wearers of any type of contact lenses.

According to a preferred embodiment, the composition of the invention is intended for topical application, advantageously by instillation in the eye(s) or by spraying the ocular surface. In other words, the composition of the invention is ocularly administered, advantageously in the affected eye(s).

The dosage is adapted to the severity of the disease but generally consists of administering one to several drops per day in the affected eye. Thus, a dosage of 10 drops per day and per eye, or even 9, 8, 7, 6, 5, 4, 3, 2 or even 1 drop per day and per eye can be considered. According to one embodiment, the composition is administered in the form of a drop

between from at least once per day to at least ten times per, advantageously from two to four times daily.

The disclosures of each and every patent, patent application, and publication cited herein are hereby incorporated herein by reference in their entirety.

Without further description, it is believed that one of ordinary skill in the art can, using the preceding description and the following illustrative examples, make and utilize the compounds of the present invention and practice the claimed methods.

## EXPERIMENTAL EXAMPLES

The invention is further described in detail by reference to the following experimental examples and the attached figures. These examples are provided for purposes of illustration only, and are not intended to be limiting.

## FIGURES

Figure 1: Ratio (%) between apoptotic cells and total cells at 8h+Overnight.
Figure 2: Antioxidant profile of different formulations relative to negative control (%).
Figure 3: Replicates mean of ratio (%) between apoptotic cells and total cells.
Figure 4: Quantification of IL8 release 24 hours post UV irradiation.

NC = Negative Control: Treated with 0,9% NaCl, Not irradiated
PC = Positive Control: Treated with 0,9% NaCl, irradiated

## EXAMPLE I- Example of a composition according to the invention

### *1. Composition*

| PRODUCT | FUNCTION | CENTESIMAL FORMULA (g/100ml) |
|---|---|---|
| Sodium hyaluronate Non-reticulated 100 < MW < 800 kDa | Lubricating and moisturizing polymer | 0.15 |
| Trehalose | Oligosaccharide | 3.00 |
| Resveratrol | Antioxidant | 0.05 |
| CREMOPHOR RH 40 | Solubilizing agent | 1 |
| Sodium chloride (NaCl) | Isotonizing agent | 0.20 |
| Trisodium citrate, $2H_2O$ 20 mM | Buffer agent | 0.588 |
| Hydrochloric acid (HCl) | pH adjustment | Up to pH 6 |
| Water ($H_2O$) | Vehicle | qsp 100 ml |

After preparation, the composition is advantageously sterilized, for example on a sterile filter at 0.2µm.

After sterilization, such a composition can be packaged in a single-dose (or single-use) vial or in a preservative free multi-dose vial such as ABAK® or NOVELIA®. The single doses are traditionally made of low-density polyethylene (LDPE) without additives.

### *2. Characteristics of the composition*

a/ Stability of the composition after 3 months in various storage conditions

The results are shown in Table 1 below:

| ANALYSIS | CHARACTERISTICS | | | | | |
|---|---|---|---|---|---|---|
| Storage conditions | 25°C 40% RH | | 30°C 35% RH | | 40°C 25% RH | |
| | Initial | 3 months | Initial | 3 months | Initial | 3 months |
| Appearance | Limpid solution, colourless and homogeneous | Limpid solution, colourless and homogeneous | Limpid solution, colourless and homogeneous | Limpid solution, slightly yellow and homogeneous | Limpid solution, colourless and homogeneous | Limpid solution, yellow and homogeneous |
| Opalescence (NTU) | 4.36 | 5.04 | 4.36 | 5.04 | 4.36 | 6.74 |
| pH | 6.10 | 6.08 | 6.10 | 6.08 | 6.10 | 6.01 |
| Osmolality (mosmol/kg) | 244 | 246 | 244 | 246 | 244 | 250 |
| Viscosity (mPa.s) | 3.6 | 3.4 | 3.6 | 3.5 | 3.6 | 3.4 |
| Resveratrol (g/100 mL) | 0.0501 | 0.0498 | 0.0501 | 0.0498 | 0.0501 | 0.0494 |
| Trehalose (g/100 mL) | 3.01 | 3.02 | 3.01 | 3.03 | 3.01 | 3.06 |
| Sodium hyaluronate (g/100 mL) | 0.147 | 0.146 | 0.147 | 0.146 | 0.145 | 0.147 |
| RH: Relative Humidity | | | | | | |

b/ Stability of the composition after a 3-month storage in a preservative-free multidose vial

The results are shown in Table 2 below:

| ANALYSIS | CHARACTERISTICS | | | |
|---|---|---|---|---|
| | Content of the vial | | Content of the extracted drops | |
| | Initial | 3 months | Initial | 3 months |
| Appearance | Limpid solution, colourless and homogeneous | Limpid solution, slightly yellow and homogeneous | Limpid solution, colourless and homogeneous | Limpid solution, slightly yellow and homogeneous |
| Opalescence (NTU) | 4.36 | 5.14 | 3.89 | 5.16 |
| pH | 6.10 | 6.03 | 6.05 | 6.00 |
| Osmolality (mosmol/kg) | 244 | 245 | 246 | 248 |
| Viscosity (mPa.s) | 3.6 | 3.8 | 3.6 | 3.6 |
| Resveratrol (g/100 mL) | 0.0501 | 0.0478 | 0.0502 | 0.0486 |
| Trehalose (g/100 mL) | 3.01 | 3.02 | 3.03 | 3.08 |

(continued)

| ANALYSIS | CHARACTERISTICS | | | |
|---|---|---|---|---|
| | Content of the vial | | Content of the extracted drops | |
| | Initial | 3 months | Initial | 3 months |
| Sodium hyaluronate (g/100 mL) | 0.145 | 0.146 | 0.147 | 0.146 |

c/ Conclusions

In conclusion, a composition according to the invention displays the following characteristics compatible with ocular administration:

- a slightly acid pH;
- an hypotonic to isotonic formulation;
- a viscosity close to human tears (about 6.5 mPa.s at 20°C).

Moreover, the stability studies show that such a composition is stable at least after 3 months. The characteristics of the composition remain unchanged after storage at room conditions. In more extreme conditions, despite a non-significant decrease in the resveratrol content as well as a slight yellowing, the composition still remains compliant.
Besides, it can stably be stored in a preservative free delivery system.

**EXAMPLE II- Study of the effectiveness of a composition according to the invention:**

*1. Cytotoxicity / irritation potential*

An initial study on human corneal epithelium (HCE) was conducted to check the cytotoxicity of a selection of tear substitutes in order to further assess their protection against superficial corneal epithelium oxidative stress damages induced by UVA+B light (290-400nm).
The preliminary cytotoxicity was performed after 4h acute exposure and after 8h exposure (repeated twice a day with the final readout the day after; 0,015J/$cm^2$) to tear substitutes using 2 assays: MTT test and TUNEL assay.
The preliminary cytotoxicity was tested *in vitro* for the following tear substitutes:

- Composition according to Example I
- Visu XL (market reference containing 0.1% Coenzyme Q10, 0.1% cross-linked hyaluronic acid (HA), 0.5% Vitamin E TPGS; pH 6.9)

on the HCE model compared to Benzalkonium Chloride (BAK) 0.1% solution (positive control) and saline solution (NaCl 0.9%; negative control).

**METHODS**

An EPISKIN reconstituted Human Corneal Epithelium (HCE) of 0,5 $cm^2$ was used for the evaluation. Epithelial human Immortalized cells (HICEC) were deposed on a polycarbonate filter and cultured at the air-liquid interface for 5 days in a chemically defined medium in order to form a structured epithelium. The inserts containing the tissues at day 5 (thickness: 55μm; 5.5 cell layers) were shipped at room temperature in a multi-well plate filled with an agarose nutrient solution in which they were embedded.
The HCEs were placed in 6 well plates with 1mL maintenance medium overnight in incubator $CO_2$ at 37°C and 90% humidity. The tissues were then used the day after, following acute 4h exposure and 8h repeated exposure and overnight protocol.
30 μL of tear substitute were applied directly on the Human Corneal Epithelium (HCE); 30 μL of saline solution and BAK 0.1% have been used as negative and positive controls, respectively.
Two exposure protocols were carried on:

- acute exposure protocol (4h): 4h exposure followed by tissues rinsing
- repeated exposure and overnight protocol (8h+overnight): twice daily exposure, with at least 4 hours between the two

treatments and readout after overnight incubation.

The protocols were performed on triplicate tissues.

At the end of the exposures, the following endpoints were investigated:

- Cytotoxicity analysis by MTT
- Immunofluorescence analysis by TUNEL Assay to detect apoptotic cells.

**VIABILITY ASSAY: MODIFIED MTT TEST**

**Principle of the method**

The method allows to evaluate a toxic event and related cytotoxicity by a colorimetric assay. Solution of MTT in balanced saline is yellowish in color. Mitochondrial dehydrogenase of viable cells cleaves the tetrazolium ring yielding blue/purple MTT crystals which are insoluble in aqueous solutions.

**Procedure**

The residual viability after treatment by MTT test was performed according to SOP M 04 with a modified protocol that includes the assessment of superficial layers.

**Data acquisition**

The Accuracy of the spectro-photometrical measurements are in the range between 0 and 2 OD: < +/-(1% +10 mOD). The Raw data of OD are directly recorded in the Microplate autoreader (TECAN INFINITE M-200) further processed by Software Magellan 6.5.

The mean of blank OD has been subtracted to every single OD value. The viability of each biological replicate has been calculated dividing the respective OD mean for the optical density average of the negative control (NC) Biological Replicates. The viability % mean of tissues treated with the same substance has been calculated.

**Acceptance criteria**

NC meets the acceptance if the mean OD negative control value of the two tissues is ≥ 1, and if the Standard Deviation value (SD) of the % viability is < 18 %.

Test substance meets the acceptance criteria if the Standard Deviation value (SD) of the cell viability % (expressed as percentage compared to NC) between the 2 tissue replicates is<18 %.

**TUNEL ASSAY**: **APOPTOTIC CELLS DETECTION**

**Principle of the method**

Cleavage of genomic DNA during apoptosis may yield double-stranded, low molecular weight DNA fragments (mono-and oligonucleosomes) as well as single strand breaks ("nicks") in high molecular weight DNA. Those DNA strand breaks can be identified by labeling free 3'-OH termini with modified nucleotides in an enzymatic reaction.

**Procedure**

Labeling of DNA strand breaks, by Terminal deoxynucleotidyl transferase (TdT), which catalyzes polymerization of labeled nucleotides to free 3'-OH DNA ends in a template-independent manner (TUNEL-reaction) has been performed by In Situ Cell Death Detection Kit (Roche, cat.11684795910) on paraffin sections following producer's instructions. The nuclei were counterstained with DAPI and mounted on microscope slides, ready to be analysed with LEICA DMi8 THUNDER imager 3D, camera K5 in fluorescence and processed with LASX 3.7.1 software. For each sample, the entire stained section has been acquired at 20X magnification (12-13 acquisitions for samples): the number of Tunel (green) positive cells and the total cells (blue, DAPI) present inside the tissue have been counted; moreover, the ratio % between positive and total cells has been evaluated.

**RESULTS**

The results are shown in Table 3 below:

| PRELIMINARY CYTOTOXICITY | MTT: Cellular viability (%) | | TUNEL Ratio of apoptotic cells in the whole tissue (%) | |
|---|---|---|---|---|
| | 4H | 8H+OVERNIGHT | 4H | 8H+OVERNIGHT |
| Negative Control (NC) | 100 | 100 | 5,34 | 7,41 |
| BAK 0,1% | 9,98 | 0,95 | n.a. | n.a. |
| Composition Example I | 90,13 | 96,23 | 6,50 | 5,27 |
| Visu XL | 88,01 | 53,96 | 5,42 | 15,34 |

n.a.: data not available

The MTT test results showed that:

- As expected, BAK 0,1% caused severe cytotoxicity at both exposures.
- Composition of Example I did not induce a significant cytotoxicity at both time points considered.
- Visu XL did not induce a significant cytotoxic effect after 4h acute exposure but after 8h followed by overnight incubation, the MTT values were borderline to the cytotoxicity cut-off (60% cell viability).

Based on the TUNEL assay and as shown on Figure 1, it is interesting to observe a reduction of the total number of apoptotic cells even in the long-term exposure by the formulation of Example I, suggesting a protective efficacy.

***2. Antioxidant activity***

***2-1 In vitro* assay**

The aim of this study was to investigate the potential antioxidant properties of different formulations, including the composition of Example I, by ABTS assay.

**METHODS**

**Principle of the method**

Upon reacting with potassium persulfate, ABTS or 2,2'-azinobis-(3-ethylbenzothiazoline-6-sulfonate) forms the ABTS.+ radical, which is blue to green in color. Adding antioxidant test items should reduce this radical and cause the mixture to discolour. The discoloration of the radical measured by spectrophotometry was proportional to the antioxidant proprieties of the test items.

The assay was performed using the *Antioxidant Assay kit* (Sigma Aldrich) according to the manufacturer instructions.

The method was standardized compared to the positive control Trolox (Sigma Aldrich).

**Test substances**

The following formulations have been tested:

- Visu XL at 3 concentrations (pure, 50% and 25% after dilution in assay buffer)
- Thealoz® duo at 3 concentrations (pure, 50% and 25% after dilution in assay buffer)
- Composition of Example I (containing 0.05% resveratrol) at 3 concentrations (pure, 50% and 25% after dilution in

assay buffer)

- Composition of Example I, but containing 0.01% resveratrol, at 3 concentrations (pure, 50% and 25% after dilution in assay buffer)
- Negative control: assay buffer
- Positive control: Trolox at 5 concentrations (23.4, 47, 94, 188 and 375 μg/ml)

**RESULTS**

The data presented relatively to the negative control following the equation 100-(OD test item/OD negative control x100) are shown on Figure 2.

**CONCLUSIONS**

Visu XL at 25%, 50% and 100% shows a mild antioxidant effect.
Thealoz® duo does not shown any antioxidant effect in the condition of the assay.
The composition of Example I tested at 25%, 50% or 100 % concentrations shows a high level of antioxidant effect, slightly higher for the composition containing 0.05% resveratrol than the one containing 0.01% resveratrol.

***2-2 In vivo* assay**

An experimental model on Human Corneal Epithelium (HCE) was performed to assess the protection offered by tear substitutes against corneal epithelium oxidative stress damages induced by UVA+UVB light (290-400nm) at the dose 125 mJ/cm$^2$ corresponding to 5 MED.
The formulation of Example I was tested compared to Visu XL (market reference containing 0.1% Coenzyme Q10, 0.1% cross-linked hyaluronic acid (HA), 0.5% Vitamin E TPGS; pH 6.9).
The UV light induced oxidative stress damages as GSH content, apoptosis and DNA damage, pro-inflammatory and cytotoxic responses were investigated 2h and 24h after 5 MED delivered directly on HCE surface.

**METHODS**

An EPISKIN reconstituted Human Corneal Epithelium (HCE) of 0,5 cm$^2$ was used for the evaluation. Epithelial human Immortalized cells (HICEC) were deposed on a polycarbonate filter and cultured at the air-liquid interface for 5 days in a chemically defined medium in order to form a structured epithelium. The inserts containing the tissues at day 5 (thickness: 55μm; 5 cell layers) were shipped at room temperature in a multi-well plate filled with an agarose nutrient solution in which they were embedded.
The HCEs were placed in 6 well plates with 1mL maintenance medium overnight in incubator $CO_2$ at 37°C and 90% humidity.

The protocols were performed on triplicate tissues.

The following series were part of the study:

- Negative control (NC): HCE treated with 30 μL PBS and stored in the dark (same time of the irradiation period)
- Positive control (IRR): 30 μL of PBS was applied on HCE surface before UV exposure. HCE were irradiated with 5 MED corresponding to 125 mJ/cm$^2$.
- Test items: 30 μL of the composition of Example I and Visu XL were applied on HCE surface before UV exposure.

HCEs were treated before UV exposure according to the following procedure: 2 treatments with 30 μL with at least 4 hours between the two applications and followed by overnight incubation.
The UVA+UVB irradiation was conducted the following day at 125 mJ/cm$^2$ by Oriel solar simulator with xenon arch lamp, Filter WG320 [mW/cm$^2$].
After UV radiation on the different HCE series, the tear substitutes were reapplied, 30 μL, and then the HCEs were incubated 2h and 24h at 37°C with 90% humidity.
The following tests were then conducted at one or both timepoints on HCE :

I. TUNEL ASSAY - IHC: apoptotic cells detection (at 24h)
II. IL-8 release by ELISA: pro-inflammatory chemokine (at 2h and 24h)
III. Toxilight assay: measure of membrane integrity and cytotoxicity by adenylate kinase release (at 2h and 24h)

**I/ TUNEL ASSAY: APOPTOTIC CELLS DETECTION**

See section II1. above.

**II/ TOXILIGHT BIO-ASSAY KIT cat. LT27-060**

**Principle of the method**

The ToxiLight™ bioassay is a non-destructive bioluminescent cytotoxicity assay designed to measure toxicity in mammalian cells and cell lines in culture. The kit quantitatively measures the release of adenylate kinase (AK) from damaged cells. The kit is based on the bioluminescent measurement of AK which is present in all cells. A loss of cell integrity, through damage to the plasma membrane, results in the leakage of a number of factors from cells cultured in vitro into the surrounding medium. The measurement of the release of AK from the cells allows the accurate and sensitive determination of cytotoxicity and cytolysis.

**Procedure**

At the end of the treatment, 20 µl of media are transferred in a 96-well plate and 100 µl of adenylate kinase (AK) Detection Reagent are added to the medium. After 5 min of incubation at room temperature, AK concentration is measured in each well.

**Data acquisition and analysis**

Luminescence data acquisition has been performed with TECAN INFINITE M-200.

**III/ ELISA QUANTIFICATION of IL-8 cat. RAB 0319**

**Principle of the method**

The ELISA (Enzyme-Linked Immunosorbent Assay) kit is an *in vitro* assay for the quantitative measurement of a target protein in biological samples. This assay employs a specific capture antibody coated on a 96 well plate. Standards and samples are pipetted into the wells and the target protein present in a sample is bound to the wells by the immobilized antibody. The wells are washed and a biotinylated detection antibody specific for the target protein is added. After washing away unbound biotinylated antibody, HRP-conjugated streptavidin is pipetted to the wells. The wells are again washed, a TMB solution is added to the wells and color develops in proportion to the amount of target protein bound. The Stop solution changes the color from blue to yellow, and the intensity of the color is measured at 450 nm.

**Procedure**

ELISA quantification of IL-8 has been performed on media cultures collected from protocol A and B treatment. Samples have been stored at -20°C. The human IL-8/CXCL8 Elisa kit has been carried out according to the instructions of the Millipore Elisa Kit (#RAB0319). Samples have been diluted 1:20 (2h) and 1:35 (24h).

**Data acquisition and acceptance criteria**

OD was acquired using Microplate autoreader (TECAN INFINITE M-200). The standard curve has been obtained by a 4 parameter logistic regression (4PL). The sensitivity range was 0.8-600 pg/ml. The minimum detectable dose of Human IL-8 was determined to be 1 pg/ml. Statistical analysis by ANOVA.

<u>**RESULTS**</u>

<u>1/ Cell membrane damage by TOXILIGHT: release of Adenylate kinase (AK)</u>

The Relative Luminescence Unit (RLU) data at 2h and 24h post UV irradiation are shown in Table 4 below:

| | AK RELEASE 2h post UV | AK RELEASE 24h post UV |
|---|---|---|
| NC | 759 ± 44 | 5108 ± 791 |

(continued)

| | AK RELEASE 2h post UV | AK RELEASE 24h post UV |
|---|---|---|
| IRR | 1415 ± 146 | 42349 ± 3616 |
| Composition of Example I | 8024 ± 910 | 1218± 486 |
| Visu XL | 6439± 304 | 48375 ± 1916 |

On HCE irradiated control, an increased release of AK at both time points compared to the not irradiated negative control has been quantified confirming the cytotoxic effect of 5MED UV.

24h post UV exposure and with a new treatment done immediately after UV, it was possible to underline a real protective effect of the composition of the invention from the UV induced damages: in fact, the product has induced a significant decrease of AK compared to IRR control and even if compared to NC, suggesting a positive protective effect on cellular membrane integrity. At this time point, Visu XL has not shown any protective effect from the UV induced damages on cells membrane.

<u>2/ ELISA quantification of IL-8</u>

The data at 2h and 24h post UV irradiation are shown in Table 5 below:

| | MEAN IL-8 RELEASE (pg/mL) 2h post UV | MEAN IL-8 RELEASE (pg/mL) 24h post UV |
|---|---|---|
| NC | 161 ± 30 | 985 ± 141 |
| IRR | 100 ± 10 | 4015 ± 744 |
| Composition of Example I | 0 | 0** |
| Visu XL | 0 | 601 ± 105** |
| ** p<0.01. Statistical analysis by one-way ANOVA with post-hoc Tukey HSD Test. | | |

At 2h post UV, the irradiation effect (IRR) has not determined a significant IL-8 release but the release was significantly increased at the 24h timepoint.

The product Visu XL blocks IL-8 release on a short timeframe only (observed only 2h post-irradiation). On the contrary, at both time points, a composition according to the invention has shown a very significant anti-inflammatory activity, where no IL-8 could be detected at least during 24h post-irradiation.

<u>3/ TUNEL staining: apoptosis</u>

The data 24h post UV irradiation are shown on Figure 3.

They reveal about 30% of apoptotic cells in the irradiated control (IRR), as well as in the presence of the Visu XL product. On the contrary, the composition of Example I (according to the invention) inhibits formation of apoptotic cells (to about 10%) and related DNA damages.

**<u>CONCLUSIONS</u>**

The data presented above confirm the antioxidant properties of the composition of Example I *in vitro* but also *in vivo.* Moreover, they show that the composition of Example I confers:

- Protection against inflammation (significant reduction of IL-8 release);
- Reduction of UV significant damages (significant lower release of adenylate kinase);
- Inhibition of apoptotic cell formation and related DNA damages (% of TUNEL positive nuclei similar to negative control).

On the contrary, the Visu XL product was effective only at 2h post-irradiation exclusively as anti-inflammatory, where significant reduction of IL-8 release was observed.

## EXAMPLE III- Comparison with other antioxidants

Whereas Example II has revealed the superiority of a composition combining the Thealoz® duo formulation and resveratrol over the market reference Visu XL (0.1% Coenzyme Q10, 0.1% cross-linked hyaluronic acid (HA), 0.5% Vitamin E TPGS; pH 6.9), it was decided to test further antioxidants in the Thealoz® duo background.

The study consisted in revealing the antioxidant power of 4 samples of eye drops by the LUCS method (AOP1) to evaluate the direct antioxidant effect (neutralization of intracellular free radicals) on living human HepG2 cells.

## METHODS

### LUCS Method (AOP1)

### Principle of the method

The LUCS method owned by AOP is based on the production of free radical species by the cells following the addition of a fluorescent biosensor targeting nucleic acids in the culture medium. The combined effect of light and the presence of the biosensor (photoinduction) leads to the production of singlet oxygen which in turn triggers the production of reactive oxygen species (ROS), a cascade of biochemical reactions that results in a positive change in the emitted fluorescence.

The LUCS test measures the ability of an antioxidant to neutralize intracellular oxidative stress. The effect is measured by the delay induced in the kinetics of the increase of the fluorescence emission. The method has been standardized on 96-well cell culture plate format to ensure high throughput and reliable statistical analysis (WO2009087229; https://www.nature.com/articles/s41598-017-18211-2).

### Procedure

The study was performed on cultured human HepG2 liver cells. The cells were respectively seeded in 96-well plates at 75,000 cells per well in complete DMEM medium in the presence of SVF and left in an incubator for 24h at 37°C/5% CO2.

Experiments were performed in DMEM medium without SVF. The cells were incubated with the different samples (8 dilutions obtained by serial dilutions in steps of 2) for 4h at 37°C/5% CO2. Two independent experiments were performed on 96-well plates, each in triplicate. After the 4h incubation, HepG2 cells were treated with the fluorescent biomarker for 1 h, then the measurement (RFU at 535 nm) was performed according to a recurrent protocol (20 iterations) of illuminations of the 96-well plate at 480 nm and fluorescence readings.

The cellular antioxidant effect index (AOP index) is calculated from the normalized kinetics according to the formula :

$$AOP\ index\ (\%) = 100 - 100\ ({}_0\int^{20} RFU_{échantillon}\ /\ {}_0\int^{20} RFU_{contrôle})$$

The dose-response curves obtained by compiling the AOP indices as a function of the log(10) of the sample concentration are processed by fit sigmoid according to the equation:

$$AOP\ index = AOP\ index_{min} + (AOP\ index_{max} - AOP\ index_{min})/(1 + 10^{(Log(EC50-CE)*HS)})$$

to calculate EC50 (concentration for which the test compound exerts 50% of the total effect or half effect), EC10 and EC90.

## SAMPLES

The samples tested in this study are:

- Sample n°1 = Thealoz® duo + TPNa (Tocopherol Phosphate sodium) 0.5%
- Sample n°2 = Thealoz® duo + TPGS VitE (Vitamin E polyethylene glycol succinate) 0.5%
- Sample n°3 = Thealoz® duo + Resveratrol 0.01%
- Sample n°4 = Thealoz® duo + Quercetin 0.01%

| | Concentration in product (g/100mL) | |
|---|---|---|
| | **(1) Thealoz duo + TPNa** | **(2) Thealoz duo + TPGS VitE** |
| NaCl | 0.201 | 0.260 |
| Tris (Trometamol) | 0.121 | 0.121 |
| Vitamin E phosphate (TPNa) | 0.500 | - |
| NaOH 2N | Ad TPNa dissolution | - |
| Vitamin E TPGS | - | 0.505 |
| Trehalose | 3.312 | 3.312 |
| Sodium hyaluronate | 0.156 | 0.156 |
| HCl 1N | Ad pH 7,2 | Ad pH 7,2 |
| Water for injection | Ad 100mL | Ad 100mL |

| | Concentration in product (g/100mL) | |
|---|---|---|
| | **(3) Thealoz duo + Resveratrol** | **(4) Thealoz duo + Quercetin** |
| Cremophor RH40 | 1 | - |
| NaCl | 0.26 | 0.2 |
| Tris (Trometamol) | 0.121 | 0.121 |
| Resveratrol | 0.01 | - |
| Quercetin | - | 0.01 |
| Trehalose | 3.34 | 3.17 |
| Sodium hyaluronate | 0.16 | 0.157 |
| HCl 1N | Ad pH 6,5 | Ad pH 7,2 |
| Water for injection | Ad 100mL | Ad 100mL |

The samples, in liquid form in amber bottles and labelled samples 1, 2, 3 and 4 were kept at 4°C in the dark until experimentation.

**RESULTS**

The results are presented in Table 6 below:

| Sample tested | AOP1 (direct effect) |
|---|---|
| (1) Thealoz® duo + TPNa | AOP index at dilution 1/64 = 63<br>AOP index at dilution 1/128 = 1 |
| (2) Thealoz® duo + TPGS VitE | AOP index at dilution 1/64 = -161<br>AOP index at dilution 1/128 = -140 |
| (3) Thealoz® duo + Resveratrol | AOP index at dilution 1/64 = 706<br>AOP index at dilution 1/32 = 926 |
| (4) Thealoz® duo + Quercetin | AOP index at dilution 1/2 = 929<br>AOP index at dilution 1/4 = 739 |

**CONCLUSIONS**

The formulations Thealoz® duo + TPNa and Thealoz® duo + TPGS VitE do not show any antioxidant activity.

Thealoz® duo + Resveratrol shows potent direct antioxidant activity by neutralizing intracellular free radicals with

antioxidant indices (AI) close to 1000 (theoretical maximum) at dilution factors ≥ 1/32.

Thealoz® duo + Quercetin exhibits potent direct antioxidant activity with antioxidant indices (AI) near 1000 (theoretical maximum) at dilution factors ≥ 1/2.

This activity appears to be more potent in this model with the formulation with resveratrol than with quercetin.

In conclusion, in the Thealoz® duo background, resveratrol has a better antioxidant effect than quercetin at the same concentration. In other words, resveratrol is the most potent antioxidant in that context.

**EXAMPLE IV- Contribution of trehalose in a composition according to the invention**

***1. Stability***

In order to study the stability of resveratrol (R) in presence of trehalose (T) or trehalose + hyaluronic acid (T+HA), the quantity of resveratrol has been determined after storage at 60°C for 7 days.

To render the test more sensitive, resveratrol was used at a concentration of around 0.08 g/100 mL of the tested formulations.

The composition of the different formulations tested, as well as the end amount of resveratrol in said formulations, are presented in Table 7 below:

| INGREDIENTS (g/100 mL or %) | Composition according to the invention (R+T+HA) | Composition without hyaluronic acid (R+T) | Composition without hyaluronic acid and trehalose (R) |
|---|---|---|---|
| Sodium hyaluronate | 0.15 | - | - |
| Trehalose | 3.00 | 3.00 | - |
| Resveratrol (initial concentration) | 0.0827 | 0.0834 | 0.0835 |
| CREMOPHOR RH 40 | 1 | 1 | 1 |
| Water ($H_2O$) | qsp 100 ml | qsp 100 ml | qsp 100 ml |
| Resveratrol (final concentration after storage at 60°C for 7 days) | 0.0789 (-4.6%) | 0.0801 (-3.9%) | 0.0778 (-6.8%) |

Said data reveal that under these conditions of storage, resveratrol is significantly less stable in the absence of trehalose (last column).

***2. Activity***

In order to evaluate the impact of trehalose on the activity of a composition according to the invention, the experiments disclosed in Example II-2-2 (antioxidant activity tested *in vivo* by IL-8 release quantification - ELISA test) have been reproduced using the formulations disclosed in Table 8 below:

| INGREDIENTS (g/100 mL or %) | P1: Composition according to the invention (R+T+HA) | P2: Composition P1 without hyaluronic acid (R+T) | P3: Composition P1 without hyaluronic acid and trehalose (R) | P4: Composition P1 without hyaluronic acid and resveratrol (T) |
|---|---|---|---|---|
| Sodium hyaluronate | 0.15 | - | - | - |
| Trehalose | 3.00 | 3.00 | - | 3.00 |
| Resveratrol | 0.05 | 0.05 | 0.05 | - |
| Same buffer system as in Example 1 | | | | |

As shown on Figure 4, 24h post-irradiation, a decrease in IL-8 release is observed in presence of trehalose alone (P4). Trehalose would then contribute, besides resveratrol, to inhibit inflammation.

## Claims

1. An ophthalmic composition comprising:

 - hyaluronic acid, or one of its salts, advantageously of a molecular weight comprised between 100 and 800 kDa;
 - an oligosaccharide, which is trehalose; and
 - resveratrol.

2. A composition according to claim 1, which is an aqueous solution.

3. A composition according to any of the preceding claims, wherein it further comprises a solubilizing agent, advantageously macrogol hydroxystearate, more advantageously polyoxyl 40 hydrogenated castor oil.

4. A composition according to claim 3, wherein the solubilizing agent represents between 0.1 and 20% (w/v), advantageously between 0.5 and 5% (w/v), more advantageously 0.5% or 1%. (w/v).

5. A composition according to any of the preceding claims, wherein it displays a pH lower than 7, advantageously comprised between 5.5 and 6.9, more advantageously equal to 6.

6. A composition according to claim 5, wherein it comprises a buffer selected in the group consisting of citrate, borate and phosphate, advantageously citrate.

7. A composition according to any of the preceding claims, wherein hyaluronic acid or one of its salts represents between 0.05 and 0.5 g in 100 ml of the composition, advantageously between 0.1 and 0.2 g/100 ml, even more advantageously 0.15 g/100 ml.

8. A composition according to any of the preceding claims, wherein the oligosaccharide represents between 0.5 and 5 g in 100 ml of the composition, advantageously between 2 and 4 g/100 ml, even more advantageously 3 g/100 ml.

9. A composition according to any of the preceding claims, wherein resveratrol represents between 0.01 and 0.1 g in 100 ml of the composition, advantageously between 0.04 and 0.06 g/100 ml, even more advantageously 0.05 g/100 ml.

10. A composition according to any of the preceding claims, wherein it is deprived of any preservative agent of the antimicrobial type.

11. A composition according to any of the preceding claims for use in the treatment of ocular diseases, advantageously dry eye disease (DED).

12. A composition for its use according to claim 11, wherein it is ocularly administered.

13. A composition for its use according to claim 11 or 12, wherein it is administered between from at least once per day to at least ten times per day in each affected eye of a human or an animal, advantageously from two to four times daily.

14. Single-use or preservative free multi-dose vial containing the composition according to any of claims 1 to 10.

## Patentansprüche

1. Augenheilkundliche Zusammensetzung, umfassend:

 - Hyaluronsäure oder eines von deren Salzen, vorteilhafterweise mit einem Molekulargewicht im Bereich von 100 bis 800 kDa;
 - ein Oligosaccharid, bei dem es sich um Trehalose handelt; und
 - Resveratrol.

2. Zusammensetzung gemäß Anspruch 1, wobei es sich um eine wässrige Lösung handelt.

3. Zusammensetzung gemäß einem beliebigen der vorhergehenden Ansprüche, wobei sie weiterhin einen Lösungsvermittler, nämlich vorteilhafterweise Macrogol-Hydroxystearat, noch vorteilhafterweise Polyoxyl-40-hydriertes Rizinusöl, umfasst.

**4.** Zusammensetzung gemäß Anspruch 3, wobei der Lösungsvermittler zwischen 0,1 und 20% (w/v), vorteilhafterweise zwischen 0,5 und 5% (w/v), noch vorteilhafterweise 0,5% oder 1% (w/v) ausmacht.

**5.** Zusammensetzung gemäß einem beliebigen der vorhergehenden Ansprüche, wobei sie einen pH-Wert von weniger als 7 aufweist, wobei dieser vorteilhafterweise im Bereich 5,5 bis 6,9 liegt, noch vorteilhafterweise gleich 6 ist.

**6.** Zusammensetzung gemäß Anspruch 5, wobei sie sie einen Puffer umfasst, welcher aus der Gruppe ausgewählt ist, die aus Citrat, Borat und Phosphat besteht, wobei es sich vorteilhafterweise um Citrat handelt.

**7.** Zusammensetzung gemäß einem beliebigen der vorhergehenden Ansprüche, wobei Hyaluronsäure oder eines von deren Salzen in 100 ml der Zusammensetzung zwischen 0,05 und 0,5 g, vorteilhafterweise zwischen 0,1 und 0,2 g/100 ml, noch vorteilhafterweise 0,15 g/100 ml ausmacht.

**8.** Zusammensetzung nach einem der vorangehenden Ansprüche, wobei das Oligosaccharid zwischen 0,5 und 5 g in 100 ml der Zusammensetzung, vorteilhafterweise zwischen 2 und 4 g/100 ml, noch vorteilhafterweise 3 g/100 ml ausmacht.

**9.** Zusammensetzung gemäß einem beliebigen der vorhergehenden Ansprüche, wobei Resveratrol in 100 ml der Zusammensetzung zwischen 0,01 und 0,1 g, vorteilhafterweise zwischen 0,04 und 0,06 g/100 ml, noch vorteilhafterweise 0,05 g/100 ml ausmacht.

**10.** Zusammensetzung gemäß einem beliebigen der vorhergehenden Ansprüche, wobei sie von frei jedweden Konservierungsstoffen vom antimikrobiellen Typ ist.

**11.** Zusammensetzung gemäß einem beliebigen der vorhergehenden Ansprüche zur Verwendung bei der Behandlung von Augenerkrankungen, wobei es sich vorteilhafter um das Syndrom des trockenen Auges (KCS) handelt.

**12.** Zusammensetzung zur Verwendung derselben gemäß Anspruch 11, wobei sie okular verabreicht wird.

**13.** Zusammensetzung zur Verwendung derselben gemäß Anspruch 11 oder 12, wobei sie zwischen mindestens einmal täglich bis mindestens zehnmal täglich in jedes der betroffenen Augen eines Menschen oder eines Tieres verabreicht wird, vorteilhafterweise zwei- bis viermal täglich.

**14.** Durchstechflasche, die zum Einmalgebrauch oder zum Mehrfachgebrauch ohne Konservierungsstoffe bestimmt ist, wobei sie die Zusammensetzung gemäß einem beliebigen der Ansprüche 1 bis 10 enthält.

## Revendications

**1.** Composition ophtalmique comprenant :

- de l'acide hyaluronique, ou l'un de ses sels, avantageusement d'un poids moléculaire compris entre 100 et 800 kDa ;
- un oligosaccharide, qui est du tréhalose ; et
- du resvératrol.

**2.** Composition selon la revendication 1, qui est une solution aqueuse.

**3.** Composition selon l'une quelconque des revendications précédentes, laquelle comprenant en outre un agent solubilisant, avantageusement de l'hydroxystéarate de macrogol, encore plus avantageusement de l'huile de ricin hydrogénée polyoxyl 40.

**4.** Composition selon la revendication 3, dans laquelle l'agent solubilisant représente entre 0,1 et 20 % (p/v), avantageusement entre 0,5 et 5 % (p/v), encore plus avantageusement 0,5 % ou 1 % (p/v).

5. Composition selon l'une quelconque des revendications précédentes, laquelle présentant un pH inférieur à 7, avantageusement compris entre 5,5 et 6,9, encore plus avantageusement égal à 6.

6. Composition selon la revendication 5, laquelle comprenant un tampon sélectionné dans le groupe constitué de citrate, borate et phosphate, avantageusement citrate.

7. Composition selon l'une quelconque des revendications précédentes, dans laquelle l'acide hyaluronique ou l'un de ses sels représente entre 0,05 et 0,5 g dans 100 ml de la composition, avantageusement entre 0,1 et 0,2 g/100 ml, encore plus avantageusement 0,15 g/100 ml.

8. Composition selon l'une quelconque des revendications précédentes, dans laquelle l'oligosaccharide représente entre 0,5 et 5 g dans 100 ml de la composition, avantageusement entre 2 et 4 g/100 ml, encore plus avantageusement 3 g/100 ml.

9. Composition selon l'une quelconque des revendications précédentes, dans laquelle le resvératrol représente entre 0,01 et 0,1 g dans 100 ml de la composition, avantageusement entre 0,04 et 0,06 g/100 ml, encore plus avantageusement 0,05 g/100 ml.

10. Composition selon l'une quelconque des revendications précédentes, dans laquelle étant privée de tout agent conservateur de type antimicrobien.

11. Composition selon l'une quelconque des revendications précédentes pour utilisation dans le traitement de maladies oculaires, avantageusement la maladie de l'œil sec (DED).

12. Composition pour son utilisation selon la revendication 11, dans laquelle elle est administrée par voie oculaire.

13. Composition pour son utilisation selon la revendication 11 ou 12, dans laquelle elle est administrée entre au moins une fois par jour et au moins dix fois par jour dans chaque œil affecté d'un humain ou d'un animal, avantageusement de deux à quatre fois par jour.

14. Flacon à usage unique ou multidose sans conservateur contenant la composition selon l'une quelconque des revendications 1 à 10.

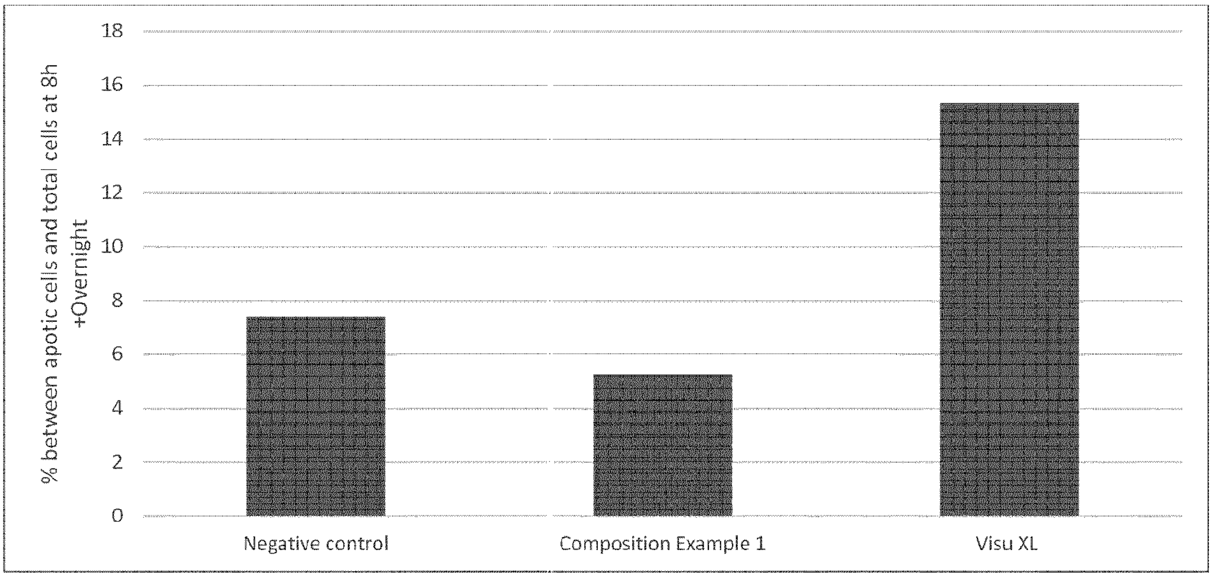
% between apotic cells and total cells at 8h +Overnight
18
16
14
12
10
8
6
4
2
0
Negative control
Composition Example 1
Visu XL

Figure 1

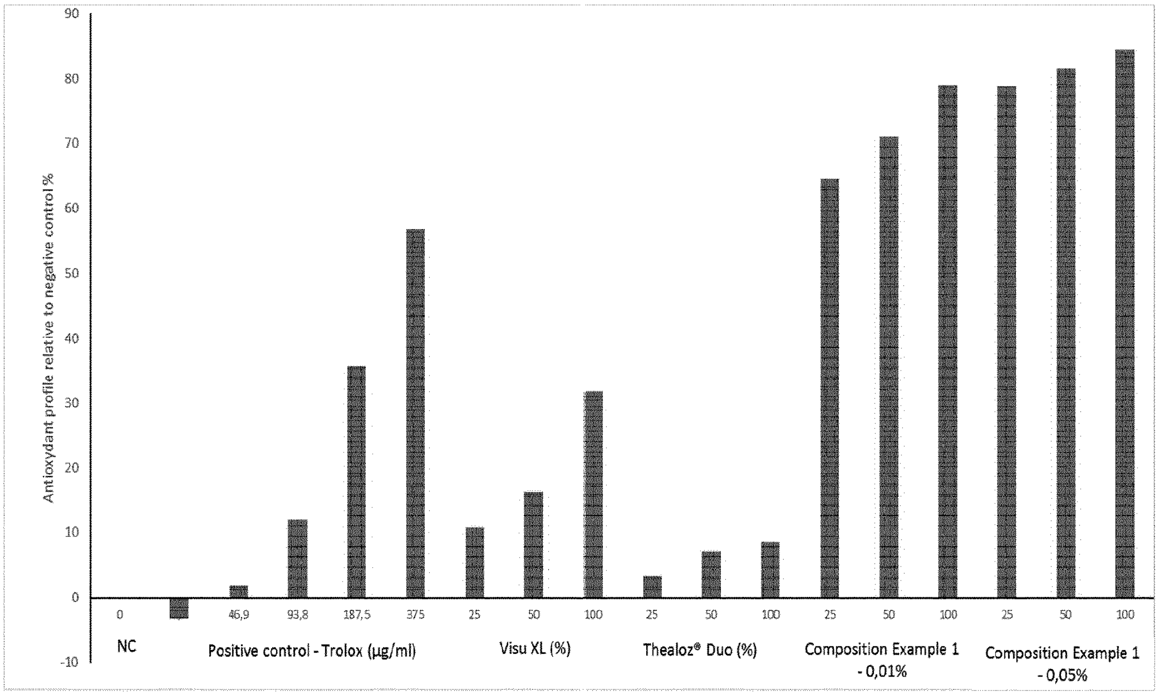

Antioxydant profile relative to negative control %
90
80
70
60
50
40
30
20
10
0
-10
0
46,9
93,8
187,5
375
25
50
100
25
50
100
25
50
100
25
50
100
NC
Positive control - Trolox (µg/ml)
Visu XL (%)
Thealoz® Duo (%)
Composition Example 1 - 0,01%
Composition Example 1 - 0,05%


Figure 2

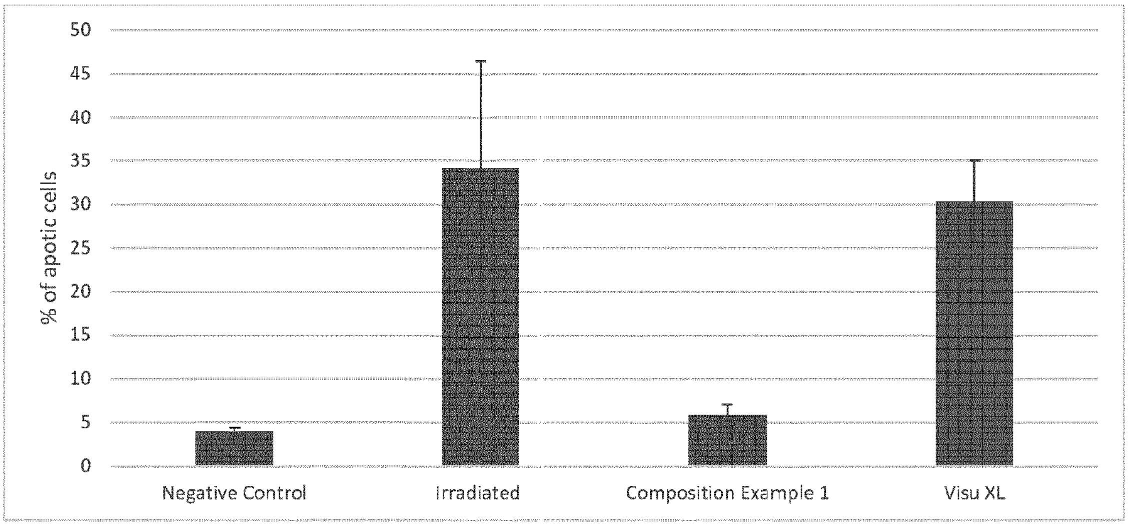


Figure 3

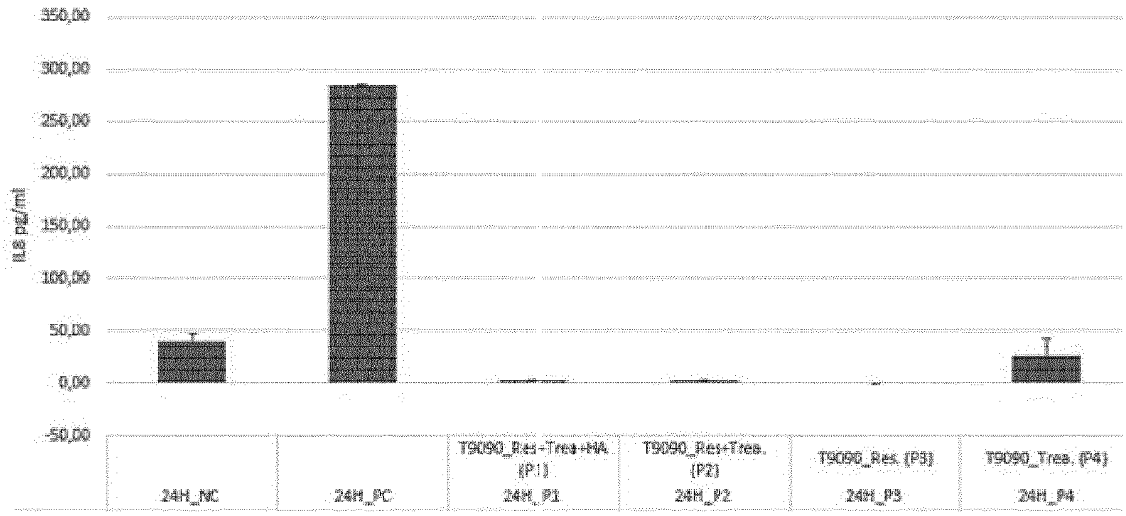


Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016151269 A
- WO 2015136186 A
- WO 2009084069 A
- WO 2009087229 A


### Non-patent literature cited in the description


- *CHEMICAL ABSTRACTS*, 61788-85-0
- *CHEMICAL ABSTRACTS*, 70142-34-6
- *CHEMICAL ABSTRACTS*, 9005-64-5
- *CHEMICAL ABSTRACTS*, 9005-67-8
- *CHEMICAL ABSTRACTS*, 9005-65-6
- *CHEMICAL ABSTRACTS*, 61791-12-6
- *CHEMICAL ABSTRACTS*, 9002-96-4